# EUROPEAN PATENT APPLICATION

(11) **EP 2 687 188 A1**
(43) Date of publication of application: **22.01.2014**
(21) Application number: 12305891.9
(22) Date of filing: 20.07.2012
(51) Int. Cl.: A61F 2/08, A61L 27/00

(54) **Artificial tendon or ligament with varying stiffness along its length**

(71) Applicant: Le Centre National de la Recherche Scientifique, 75794 Paris Cedex 16 (FR); ASSOCIATION POUR LA RECHERCHE ET LE DEVELOPPEMENT DES METHODES ET PROCESSUS INDUSTRIELS (ARMINES), 75272 Paris Cédex 06 (FR)
(72) Inventor: Cantournet, Sabine, 92170 VANVES (FR); Corte, Laurent, 75013 PARIS (FR); Ku, David N., DECATUR, GA 30030 (US); Cherkaoui, Mohammed, 57535 MARANGE SILVANGE (FR); Bach, Jason, 57070 METZ (FR)
(74) Representative: Bourgouin, André

(57) **Abstract**

Tendon or ligament with an elongated body comprising a flexible median part and at least one end part showing a different tensile stiffness from that of the median part, said body having a fibrous structure formed from biocompatible fibers having a diameter superior to 0.1 μm, said fibers forming the body of the device wherein the median part comprises a tubular cage placed around a central core comprising fibers.

## Description

### FIELD OF THE INVENTION

The present invention relates to an elongated fibrous device for use as tissue repair.

### BACKGROUND OF THE INVENTION

Hundreds of thousands of anterior cruciate ligaments (ACL) are torn every year and this trend has been increasing with the rise of participation in sports in the general population and in particular in females and older participants. For young and/or athletic active individuals the standard care is based on ligament reconstruction. Several replacement tissues can be envisaged using either grafts (auto, allo and xeno) or artificial materials. Xenografts, ligaments from other animals, and allografts from cadaveric human tissue are possibilities that overcome the need to autologous tissues and avoid the risk of donor-site morbidity. However, their use poses several issues including risks of disease transmission, graft rejection and inflammation. Moreover, in the case of allografts, the supply is so small that the market demand can never be met from this source. Autograft tissues extracted from the patellar tendon, quadriceps tendon-patellar bone or the hamstring tendons are currently the most common sources of grafts for ACL reconstruction. Yet this therapy relies on the extraction of healthy tissue which implies risks of donor-site morbidity, an initial low strength, a high probability of rupture at the initial stages and a long and painful recovery period. The use of artificial prosthetic ligaments as an alternative to autografts could bring substantial improvements in the existing reconstruction therapies.

Several prosthetic devices for ACL replacement have been made over the past thirty years using a wide range of materials. The materials which have been considered for these devices including polyester (Stryker Dacron® ligament prosthesis, Leeds-Keio), polytetrafluoroethylene and fluoropolymers (Gore-Tex®), carbon fibers, polyethylene, nylon and polystyrene. However none of these artificial ligaments have demonstrated positive long term results *in vivo.* Failures of previous devices mostly originate from mechanical failures or from a lack of biocompatibility. Mechanical failures include i) rupture caused by wear, fatigue or severe loading in the knee and ii) laxity in the joint after creep of the prosthetic ligament or loosening of the fixation element in the bone. Biocompatibility issues primarily manifest as immunogenic particulation leading to chronic synovitis. Due to high incidence of such problems, most if not all the previous artificial ligaments have been withdrawn from the commercial market. For example, no such devices are currently approved for clinical use by the Food and Drug Administration of the United States of America (FDA).

Over the last decades, statistics have been collected that confirm the poor long term efficacy of existing artificial ligaments. A study of 855 artificial cruciate ligaments over a 15-year period found that there was 40-78% failure rate (Fu F. H. et al. American Journal of Sports Medicine, 2000, 28(1), 124-130). Another report found that around 80% of knees which had been reconstructed using Dacron prosthetic ligaments had developed significant osteoarthritic symptoms at a 9 year follow up (Fu F. H. cited above). Similarly, a study of 268 patients revealed that Gore-Tex® anterior cruciate ligament prosthesis yielded a failure rate of 42% with case of effusions, rupture and strong loosening (George M.S. et al, American Journal of Sports Medicine, 2006, 34(12), 2026-2037). Overall, complication rates for artificial ligament operations are of the order of 40-50%, which is much higher than the rate with autologous and allogenic ligaments.

As an alternative to non-biodegradable artificial ligaments, research efforts are being carried out to develop tissue engineered (TE) ligaments for which a biodegradable scaffold first replaces the native ligament and is progressively replaced by a new reconstructed living tissue. Several systems have recently been designed using silk, collagen or polylactic acid biodegradable fibers (Freeman J. W. et al. Journal of Biomechanics, 2010, doi: 10.1016/j.biomech. 2010.10.043). Nevertheless, the management of cell sourcing as well as the control of scaffold degradation while ensuring proper mechanical properties remain unsolved issues that still need to be addressed before clinical use.

Most of the patents deal with the methods of attachment or fixation design to attach an artificial ligament to the bones of a joint. Recent examples are given below.

FR 2 700 111 relates to an artificial ligament consisting of a fixed section and a moving sleeve which form two separate ligaments of the same or different lengths, joined together so that they can slide relative to one another. The two ligaments can be made from plaited, woven or knitted fibres of the same or different materials, with their ends joined together by a thermo-shrink material or a supple adhesive. The outer ligament can be made with sections of reduced resistance which allow its length to be varied. The ligaments can be made from Dacron® (RTM) or other synthetic fibres, or from natural cellulose fibres which are treated to make them biocompatible.

US 2003/114929 discloses a prosthetic ligament including a cord of thermotropic liquid crystal filaments. Preferably the cord is a string or thin rope made by several strands braided, twisted, or woven together. Strands are, preferably, made of a multi-filament thread.

US 5800543 relates to an artificial ligament device comprising a plurality of tows of biocompatible material (for example polyester) secured side-by-side in a flat elongate array by braiding, the tows being looped back at one end of the device to form an eye, the flat lengths adjoining the eye being secured to each other side-by-side by stitching, the tows around the eye being grouped together and whipped, and lashing being applied around a base of the eye.

WO 2009/047767 provides a ligament prosthesis having a first end and a second end, comprising a first load bearing element and a second load bearing element, the first and second load bearing elements differing in one or more mechanical properties and being arranged in the prosthesis in series. The load bearing elements may be made from an alloy and, in order to protect nearby organs and tissues from abrasion from the prosthesis and vice versa, may be contained in a sleeve made from biodegradable polymers such as poliglecaprone, polyglycolic acid, polylactic acid, polydioxanone, or co-polymers of the aforementioned polymers.

WO 2009/113076 provides a ligament prosthesis having an undeployed configuration and a deployed configuration. The prosthesis has a resistance to tension in the undeployed configuration that is less than its resistance to tension in the deployed configuration. In the deployed configuration, the prosthesis is capable of twisting and bending. In one embodiment, the prosthesis has a meshwork of filaments woven, knitted or braided into a slender cylinder. The prosthesis may be used to replace an anterior or posterior cruciate ligament.

WO 98/22046 discloses a "free strand" ligament that is naturally self-convoluted between the two ends of the intra-articular median part.

WO 89/01320 discloses a soft tissue prosthesis comprising a tubular braid having regions of stiffness and elasticity provided by stiff and elastomeric components disposed within a channel enclosed by the tube and/or within the braid, and means defining a transition region for minimizing relative motion among the braid components, the stiff components and the elastomeric components.

US 2009/0306775 discloses prosthetic ligaments and tendons comprising ligament-or tendon-mimicking nanofibers and methods of making such nanofibers and prosthetic ligaments and tendons

With the foregoing disadvantages of the prior art in mind, it appears that there is a need for a device which i) is biocompatible on the short and long term, meaning after years of implantation *in vivo,* ii) reproduces closely the non-linear elastic mechanical behaviour of native ligaments and tendons including the stiffness and the toe-region, iii) ensures ultimate tensile stress (strength) and ultimate tensile strain that are safe with respect to the patient's activity. The proper assembly of biocompatible fibers could address these requirements.

A non-biodegradable device that can be installed *in-vivo* to repair a ligament or tendon, that is biocompatible and that reproduces closely the mechanical properties of native ligaments and tendons as well as ensures the appropriate values of tensile strength and ultimate tensile strain required for ligament or tendon replacement is needed.

The inventors think that a novel type of device, in particular an artificial ligament or tendon, which can be placed and fixed in a patient at the location of a ligament or a tendon, and which is made of a biocompatible fibrous structure reproduces closely the tensile mechanical response of native ligaments or tendon with values of ultimate tensile stress and ultimate tensile strain that are appropriate for ligament or tendon repair. It can be placed in the patient using minimally invasive techniques. Unlike autograft techniques, this device does not necessitate the sacrifice of healthy tendons or ligaments from the patient.

In international application PCT/EP2012/050917 filed 20 January 2012 claiming the priority of International application N° PCT/IB2011/000312, not yet published, inventors of the present application have disclosed and claimed a biocompatible device in the form of an elongated body comprising at least a flexible median part between two end parts, said body having a fibrous structure formed from biocompatible hydrogel forming fibers, said hydrogel fibers forming the body of the device.

### DESCRIPTION OF THE INVENTION

The inventors have now realized that new and improved elongated fibrous devices can be used as tissue repair and especially as prosthetic devices for tendon or ligament especially ACL replacement.

It is therefore a first object of the invention to provide a biocompatible device in the form of an elongated body comprising a flexible median part and at least one end part showing a different tensile stiffness from that of the median part, said body having a fibrous structure formed from biocompatible fibers most preferably having a diameter superior to 0.1 µm, said fibers forming the body of the device wherein the median part comprises a tubular cage placed around a central core comprising fibers.

The words "terminal end parts" can also be used in place of "end parts" but the language "end parts" is preferred.

The words "yarns" and "threads" are synonymous.

The words "cage" and "net" are also synonymous for the interpretation of the present application.

According to the invention, the word "biocompatible" means that the materials used for making the device and the device itself elicit little or no immune response in a given organism, or is able to integrate with the tissue.

According to the invention, the term braiding angle for the tubular cage is defined as the angle formed by the fiber forming the cage and the long axis of the device.

According to the invention, the structure of the invention is defined as having fibers as elementary units.

It is understood that similar structures and equally efficient devices can be obtained using threads (yarns) or larger strands as elementary units instead of fibers.

Consequently, the words "threads (yarns)" can be substituted for the word fibers in the present application.

The biocompatible fibers that can be used to make the devices according to the invention are selected from hydrogel forming fibers, biocompatible carbon fibers or metal fibers like titanium and titanium alloy fibers or polymeric fibers selected from the group consisting of polyethylene (PE), polypropylene (PP), polyamides (PA), polycarbonates (PC), polyurethanes (PU), polyurethane urea, polyesters like polyethylene terephtalate (PET), polyfluoropolymers like polytetrafluoroethylene (PTFE), polyacrylates like polymethyl methacrylate (PMMA), polyethylene glycol (PEG), poly-L-lactic acid (PLLA), poly-G-lactic acid (PGLA), poly-caprolactone (PCL), polyglycolide, polysaccharides like cellulose, hyaluronic acid, proteins like elastin, silk, collagen and from blends or copolymers obtained with polymers from this group.

The diameter of the fibers that are used can vary according to various parameters like the nature of the polymer used, the type of tendon or ligament replacement envisaged. In a most preferred embodiment, all fibers used have a diameter superior to 0.1 µm. Preferably, the fibers forming the boby of the device have a diameter superior to 1 µm

The devices according to the invention can preferably be used in the replacement of tendons when they have only one end part and in the replacement of ligaments when they have two end parts.

As will become apparent below, in a preferred embodiment of the invention, the device of the invention does not have any marked "transition region" or binding means between the median part and the at least one end part.

In a preferred embodiment of the invention, the device of the invention does not have any transition region aiming at minimising the relative motion of the various components of the device or binding means between the median part and the at least one end part.

In another preferred embodiment, the present invention consists in a device as defined above and is in the form of an elongated body comprising a flexible median part and two end parts showing a different tensile stiffness from that of the median part.

These devices are used preferably in the ligament replacement therapy, in particular in the ACL replacement.

A further object of the present invention is a biocompatible device in the form of an elongated body comprising a flexible median part between two end parts showing a different tensile stiffness from that of the median part.

A further object of the present invention is a device according in the form of an elongated body comprising a flexible median part and two end parts showing a different, preferably higher tensile stiffness from that of the median part.

Besides the structure, the nature of the materials is central to the mechanical and biological properties of the implant. Polymer hydrogels constitute relevant materials in that respect.

In another preferred embodiment, the present invention consists in a device as defined above wherein at least part of the biocompatible fibers forming the central core of the flexible median part of the device are hydrogel forming fibers.

The words "hydrogel fibers", can also be used in place of "hydrogel forming fibers" but the language "hydrogel forming fibers" is preferred. The word "forming" can also be inserted in the terms "hydrogel matrix", "hydrogel polymers".

Hydrogels, also called aquagels, are hydrophilic macromolecular networks that can absorb water and swell without dissolving at least temporarily. Depending on the physicochemical properties of these networks, levels of water absorption can vary greatly from about 10% to thousand times their dry weight. An important characteristic of hydrogels is that they can possess a water content and a molecular structure that mimics that of living tissues. These features confer them biocompatibility, lubricity, rubbery elasticity and possibly biodegradability, which are of interest for biomedical applications and more particularly tissue replacement. Examples of hydrogel forming polymers that are relevant for biomedical applications are polyvinyl alcohol, polyethylene-glycol, polysaccharides, polylactic acids and their copolymers.

US 5981826 provides a poly(vinyl alcohol) hydrogel construct having a wide range of mechanical strengths for use as a human tissue replacement. It may be especially useful in surgical and other medical applications as an artificial material for replacing and reconstructing soft tissues in humans and other mammals. Soft tissue body parts which can be replaced or reconstructed by the hydrogel include vascular grafts, heart valves, esophageal tissue, skin, corneal tissue, cartilage, meniscus, and tendon. However, the reported tensile modulus of elasticity for the so-prepared material is less than 1MPa, which is too low as compared to the ultimate tensile stress of ligaments and tendons, which is greater than 100MPa.

WO/2006/102756 relates to a hydrogel exhibiting anisotropic properties which is poly(vinyl alcohol) produced by preparing a solution of poly(vinyl alcohol) with a preselected concentration, thermally cycling the solution by freezing and thawing, stretching the hydrogel and thermally cycling the hydrogel at least one more time. Said anisotropic hydrogel is used for soft tissue replacement selected from vascular vessels, coronary arteries, heart valve leaflets, heart valve stent, cartilage, ligaments and skin. However, the ultimate tensile stress for the so-prepared materials do not exceed 0.4MPa, which is too low as compared to the ultimate tensile stress of ligaments and tendons, estimated in the range 30-50MPa.

WO/2001/017574 discloses a hydrogel intended for orthopedic applications wherein the tissue is selected from the group consisting of bone, cartilage, meniscus, bursa, synovial membranes, tendons, ligaments, muscle and vertebral disks. Like for WO/2006/102756, the ultimate tensile stress for the preferred material is about 8MPa, which is too low for the replacement of most ligaments and tendons.

JP4141178 discloses an artificial tendon and an artificial ligament consisting of polyvinyl alcohol fiber with a tensile strength of 12g/d or larger and a tensile breaking elongation of 6% or smaller which is much less than the devices of the present invention. In a preferred embodiment in the device according to the invention the central core of the flexible median part of the device comprises preferably further to hydrogel fibers, biocompatible fibers selected from biocompatible carbon fibers or metal fibers like titanium and titanium alloy fibers or polymeric fibers selected from the group consisting of polyethylene (PE), polypropylene (PP), polyamides (PA), polycarbonates (PC), polyurethanes (PU), polyurethane urea, polyesters like polyethylene terephtalate (PET), polyfluoropolymers like polytetrafluoroethylene (PTFE), polyacrylates like polymethyl methacrylate (PMMA), polyethylene glycol (PEG), poly-L-lactic acid (PLLA), poly-G-lactic acid (PGLA), poly-caprolactone (PCL), polyglycolide, polysaccharides like cellulose, hyaluronic acid, proteins like elastin, silk, collagen and from blends or copolymers obtained with polymers from this group.

A further object of the present invention is a device wherein the hydrogel forming fibers are made of polyvinyl alcohol having preferably a water absorption higher than 5% and preferably comprised between 10 and 100% in weight.

In a preferred embodiment, in the device of the invention the hydrogel forming fibers are made of polyvinyl alcohol preferably having a water absorption higher than 10% and preferably comprised between 10 and 80% in weight.

According to the invention, the word "fibrous" means that the body of the device contains preferably at least 1% in weight of products of fibers Most preferably, the body of the device contains at least 10% in weight of products of fibers

According to the invention the flexible median part reproduces the behavior of the natural ligament or tendon whereas at least one end part is designed to be fixed on a bone.

According to the invention, the body may be formed by any techniques known from the one skilled in the art, for example by simple assembly of the fibers keeping them individual, by assembly of the fibers into threads or strands, by braiding, by knitting or by weaving.

According to the invention, the device is advantageously an artificial ligament used for repairing any ligament in animals, in particular non human mammals or humans. Ligaments which may be repaired may be selected from the followings: head and neck ligaments (cricothyroid ligament, periodontal ligament, suspensory ligament of the lens), wrist ligaments (palmar radiocarpal ligament , dorsal radiocarpal ligament, ulnar collateral ligament, radial collateral ligament), shoulder ligament (rotator cuff), knee ligament (anterior cruciate ligament (ACL), lateral collateral ligament (LCL), posterior cruciate ligament (PCL), medial collateral ligament (MCL), cranial cruciate ligament (CrCL) - quadruped equivalent of ACL, caudal cruciate ligament (CaCL) - quadruped equivalent of PCL, patellar ligament).

Anterior cruciate ligament (ACL) is the most likely ligament to be replaced by the device of the invention.

According to the invention, the device is advantageously an artificial tendon used for repairing any tendon injury and in particular in the most common areas in the body affected by tendon injury. These are in particular the biceps tendon, the patellar tendon, the Achilles tendon or the rotator cuff tendon.

In another embodiment, in a device according to the invention the core of the flexible median part is made of braided, twisted, knitted, woven or longitudinally disposed fibers.

In another advantageous embodiment of the invention the tubular cage is made of fibers with a Young's modulus greater than 100MPa, preferably greater than 500MPa, made of polyethylene, preferably UHMWPE, polypropylene (PP), polyurethane (PU), polyesters like polyethylene terephthalate (PET), polyamide (PA), polyfluoropolymers like polytetrafluoroethylene (PTFE), non-polymeric fibers, preferably metallic fibers or a mixture of different fibers.

In an advantageous embodiment of the invention, the tubular cage is made of hydrogel forming fibers.

In an advantageous embodiment of the invention, the fibers (or yarns) that form the tubular cage are woven, diamond braided or helicoidally wrapped in one or several layers around the central core of the flexible median part of the device.

In a particular embodiment of the invention, the layers forming the tubular cage have different braiding angles, preferably where the braiding angle decreases from the innermost to the outermost layer.

In a particularly advantageous embodiment of the invention the fibers or yarns constituting the tubular cage are diamond braided or wrapped helicoidally with an angle comprised between 5 and 85°, preferably between 10 and 80°.

In a particularly advantageous embodiment of the invention the at least one end part, preferably two end parts, are placed adjacently to at least one of the ends, preferably each of the ends of the median part and are made of swellable and porous braided, twisted, woven or knitted tunnel shaped sections.

In a particularly advantageous embodiment of the invention the biocompatible fibers forming the fibrous structure of the elongated body of the device are continuous.

In a particularly advantageous embodiment of the invention the woven, diamond braided or helicoidally wrapped fibers that form the tubular cage placed around the median part are continuous.

In an advantageous embodiment of the invention the continuous hydrogel forming fibers forming the body of the device are braided, twisted, knitted, woven or longitudinally disposed in the median part of the device and are braided, twisted, woven or knitted to form the tunnel shaped sections in each of the two end parts of the device.

In an advantageous embodiment of the invention the continuous fibers that are woven diamond braided or helicoidally wrapped to form the tubular cage placed around the median part of the device are braided, twisted, woven or knitted with the fibers in the central core to form the tunnel shaped sections in each of the two end parts of the device.

A further object of the present invention is a device wherein the two end parts show a higher tensile stiffness than that of the flexible median part.

Another object of the present invention is a device wherein the two end parts show a higher bending stiffness than that of the flexible median part.

A further object of the present invention is a device wherein the median part shows an ultimate tensile strain greater than 6%, 6 to 100%, more advantageously from 10 to 80%.

A further object of the present invention is a device wherein the two end parts show a tensile stiffness that is from 10% to 100 times in particular 10%, 50%, 2 or 5 times higher, than that of the flexible median part.

A further object of the present invention is a device wherein the end parts are placed adjacently to each of the ends of the median part and are made of swellable, porous and braided, twisted, woven or knitted sections. The end parts sections may be permeable.

A further object of the present invention is a device wherein the swelling of the end parts is less than 40%, preferably less than 20% in weight and the porosity is greater than 1%.

The swelling of the device is measured according to standard procedures.

Swelling is the property of a device whereby the volume increases upon exposure to water.

Porosity is the property of a device whereby open spaces or pores exist without polymeric material. Cells can migrate into the spaces or pores contained in the device. Porosity is defined as the fraction of the volume of open spaces or pores over the total volume.

In some of the devices according to the invention, the flexible median part has a linear elasticity.

In some of the devices according to the invention, the flexible median part has a non linear elasticity.

The invention concerns more particularly a device having a median part whose tension stress-strain curve shows a low stiffness region at the lowest levels of tensile strain and a higher stiffness region at greater levels of strain.

In some of the devices according to the invention, the flexible median part has a linear elasticity.

In an advantageous embodiment, the tensile strength and the ultimate tensile strain of the device defined above are measured after equilibration in water or bodily liquids, until weight is stable, typically a few hours at 20°C. The same conditions also apply to the measure of the ultimate tensile load, tensile stiffness, tensile modulus and flexural modulus.

The device according to the invention shows a tensile strength between 10 and 200MPa, advantageously between 15 and 50 MPa (corresponding to an ultimate load between 1100 and 4000N for a ligament with a diameter of 1cm) and an ultimate tensile strain between 10 and 100%, advantageously 15-80%.

In an advantageous embodiment, the device according to the invention shows an ultimate tensile load between 30 and 60000N, advantageously comprised between 300 and 4000N and an ultimate tensile strain comprised between 10 and 100%, advantageously between 15 and 80%.

Another aspect of the invention concerns a device as defined above showing a tensile strength greater than 10 MPa, advantageously comprised from 15 to 50MPa.

A device according to the invention can advantageously in the flexible median part show in the 0 to 5% strain range a tensile modulus of 1 to 500 MPa in particular 1 to 10, 10 to 40, 40 to 100, 100 to 150, 150 to 300, 300 to 500 MPa.

A device according to the invention can also advantageously in the flexible median part show in the 10 to 15%. strain range a tensile modulus 1 to 500 MPa in particular 1 to 10, 10 to 40, 40 to 100, 100 to 150, 150 to 300, 300 to 500 MPa.

A further object of the present invention is a device wherein the flexible median part shows in the 0 to 5% strain range and/or in the 10 to 15%. strain range a tensile modulus of 1 to 500 MPa in particular 1 to 10, 10 to 40, 40 to 100, 100 to 150, 150 to 300, 300 to 500 MPa.

A device according to the invention wherein the median part shows in the 10 to 15% strain range a tensile stiffness comprised from about 10 to 500 N/%, preferably from about 20 to about 300 N/%.

A device according to the invention can advantageously show an ultimate tensile load greater than 30 N advantageously comprised between 300 and 4000N

A device according to the invention can advantageously show a bending stiffness from 10 to 100 MPa.

A device according to the invention wherein the median part shows a flexural modulus from 0.1 to 200 MPa advantageously comprised between 1 and 100 MPa.

According to the invention, the device is advantageously made of continuous fibers. The threads used to make the device can also be continuous.

By continuous fibers it is meant that the fibers are prolonged and span from one end of the device to the other end without interruption. Preferably, a type of fiber that covers the entire dimension of a part without a break or interruption is used.

In an advantageous embodiment of the invention, the core of the flexible median part is made of braided, twisted, knitted, woven or longitudinally disposed fibers.

In a preferred embodiment of the invention, a device according to the invention has end parts that are made of braided, twisted, woven or knitted tunnel shaped sections that contain at least one osseointegration promoting substance and/or has holes at each end of the section or on the side of it to allow injection of materials into the space inside the tunnel.

A device according to the invention is **characterized in that** at least one of the end parts is filled with a substance, preferably bone mulch or bone cement or a mineral filler preferably a bioactive glass or hydroxyapatite.

A device according to the invention has end parts that are coated or impregnated with one or several osseointegration-promoting substances, preferably calcium phosphate and/or calcium sulfate.

In a device according to the invention, at least one end part, preferably two end parts are filled, coated and/or impregnated with a substance, preferably selected from bone mulch or bone cement or a mineral filler preferably a bioactive glass or hydroxyapatite and/or with one or several osseointegration-promoting substances, preferably calcium phosphate and/or calcium sulfate.

The term bioactive glass is well known in the art. These materials usually contain Ca-phosphates and or Ca-sulphates. CaO, P₂O₅ SiO₂ and Na₂O are typical constituents for bio elements.

Examples of commercially available materials contain :
46.1 mol% SiO2, 26.9 mol% CaO, 24.4 mol% Na2O and 2.5 mol%P2O5
60 mol% Si02, 36 mol% CaO and 4 mol% P2O5.
70 mol% SiO2, 30 mol% CaO.

Other biocompatible materials like porcelain, alumina, zirconia can also be used.

In a preferred embodiment of the invention, device according to the invention, the hydrogel forming fibers are made of polyvinyl alcohol.

In a preferred embodiment of the invention, the hydrogel forming fibers are made of polyvinyl alcohol having a water absorption higher than 10% and preferably comprised between 10 and 80% in weight.

According to the invention any biocompatible hydrogel known from the one skilled in the art may be used if the moisture content is comprised between 10 and 80%. A suitable hydrogel is, for example, the PVA hydrogels disclosed in US 5 981 826, or sold by Salumedica LLC. Commercial hydrogel forming fibers can be used like PVA fibers Solvron® sold by Nitivy Company Ltd.

Preferably, the hydrogel forming fibers are made of polyvinyl alcohol fibers having a linear mass density of 8.10⁻⁵ to 2500 deniers preferably 45 deniers and a tensile strength greater than 10g/d preferably 44g/d .

Fibers having a diameter superior to 0.1 until µm made by electrospinning or fibers having a diameter from 1 to 500 µm made by conventional methods can be used.

It is well in the knowledge of the one skilled in the art to adjust the number of fibers or threads to the size and density of the fibers. For instance, if very fine fibers like those obtained by electrospinning (typically on the micro or nano scale) are used the skilled artisan will use many more fibers to make the device according to the invention.

In an advantageous embodiment according to the invention, the fibrous structure may also contain other type ofbiocompatible fibers assembled with the hydrogel fibers, for example biocompatible carbon fibers or metal fibers like titanium and titanium alloy fibers or polymeric fibers selected from the group consisting of polyethylene (PE), polypropylene (PP), polyamides (PA), polycarbonates (PC), polyurethanes (PU), polyurethane urea, polyesters like polyethylene terephtalate (PET), polyfluoropolymers like polytetrafluoroethylene (PTFE), polyacrylates like polymethyl methacrylate (PMMA), polyethylene glycol (PEG), poly-L-lactic acid (PLLA), poly-G-lactic acid (PGLA), poly-caprolactone (PCL), polyglycolide, polysaccharides like cellulose, hyaluronic acid, proteins like elastin, silk, collagen and from blends or copolymers obtained with polymers from this group.

More preferably, the fibrous structure may also contain other types of biocompatible fibers assembled with the hydrogel fibers, for example fibers made in one or several of the following polymers can be used: Polyethylene (PE), Poly-L-lactic acid (PLLA), Poly-G-lactic acid (PGLA), Poly-caprolactone (PCL), Silk, Polyesters, Polyethylene terephtalate (PET), Polytetrafluoroethylene (PTFE), PLGA, Ti wires fibers assembled with the hydrogel forming fibers.

A device according to the invention is **characterized in that** the body is partly or totally coated or entrapped in a hydrogel matrix.

According to the invention, the diameter of the device is similar to the one of the natural part to be repaired; for example in the case of an artificial ligament for repairing a human knee ligament the diameter of the device is comprised between 2 mm and 2 cm, advantageously comprised between 5 and 10 mm. The length of the body is also similar to the one of the natural ligament. For the case of the human knee ligament, it is comprised between 0.5 and 5 cm and the length of the whole ligament comprising the median part and the end parts is comprised between 5 and 25 cm, advantageously between 10 and 20 cm, more advantageously is equal to about 15 cm. The choice of the diameter and of the length of the device is well in the hand of the one skilled in the art who may adapt said parameters to the type of ligament or tendon he wishes to repair.

According to the invention, the device may further contain anchoring systems at least at one end part which may be any system known from the one skilled in the art, like for example a hook, a screw, a buckle, a bone anchor, an interference screw, a cross pin or a suture button. It may also be an eye spliced at said end of the device.

According to the invention in the device the hydrogel forming fibers forming the body of the device can be cross-linked.

In an advantageous embodiment according to the invention, either the fibers, or the assembly of fibers (threads, strands, braid, knit fabric or woven fabric) or the whole body or both of them or the three are entrapped in a hydrogel matrix or coated by a hydrogel, said hydrogel being the same or being different from the hydrogel used for the fibers. Said coating or matrix may contain mineral fillers imparting better anchoring on the bone like calcium phosphates (hydroxyapatite and the like) or may be swollen by water in situ in order to have better anchoring on the bone.

Then in an advantageous embodiment of the invention, the anchoring system may be the hydrogel coated or embedded end parts of the device themselves.

It is to be noted that the terms embedded and entrapped are usually synonymous.

Where coating by a hydrogel or embedding in a hydrogel matrix is performed on the fibers or strands, then it is performed prior to assembly of the body. Said matrix or coating plays several roles:
i) It better redeploys the efforts between fibers
ii) It lubricates the contact between the device and surrounding tissues thus diminishing the risks of inflammation and of failure by wear,
iii) It provides additional stiffness to the end parts in order to reduce deformation in the bone tunnels and favor adhesion with bone.
iv) According to the properties of the hydrogel, it is used either to prevent cellular adhesion on the median part of the body or to reinforce the bone-device interface and accelerates the osseous integration of the end parts.

The device may have any form suitable for repairing the corresponding natural part. It is generally elongated in shape and the anchoring system at at least one end of the device is adapted for attachment to a musculo-skeletal tissue such as bone.

In an advantageous embodiment of the invention, the device has three parts: a central part and two extreme parts. In the case of a cruciate ligament the extreme parts are called respectively tibial and femoral part and the central part is called ligamentous part.

In an advantageous embodiment according to the invention, the fibers may be assembled to threads, and the strands are strands of threads. The strands may be twisted in the opposite direction of the twist of the threads to gain a greater stiffness and cohesion of the structure.

In another advantageous embodiment of the invention, the fibers are oriented in a direction of loading of the prosthetic ligament.

In another advantageous embodiment of the invention, the fibers may be assembled to threads, and the threads are oriented in a direction of loading of the prosthetic ligament.

In another advantageous embodiment the assembly pattern can be different for each part. For example, where the fibers are assembled into strands, strands can be twisted in the central part and braided in the end-parts.

The fibers themselves or the coating or both may contain at least one compound selected from the group consisting of growth factors, drugs like anti-inflammatory drugs or a mineral filler like calcium phosphates (hydroxyapatite and the like).

In an advantageous embodiment, the stiffness of the device of the invention is comprised from about 10 to 500 N/%, preferably from about 20 to about 300 N/%.

An annealing step can preferably be performed at a temperature comprised between 130 and 190° for 10 minutes to one hour.

The device according to the invention may be prepared by any process known from the one skilled in the art. The process shall permit the modification of each part of the body separately.

Consequently another object of the invention is a process for preparing a device according to the instant invention with steps comprising:
a) assembly of fibers to form the body,
b) impregnation of the fibers, the assembly of fibers or a part or the whole body of the device in a solution of hydrogel forming polymer or a solution of monomer or oligomer to coat said fibers, or said strands or said assembly of fibers or said body or to embed them in a hydrogel matrix, followed in the case of a solution of hydrogen forming monomer or oligomer by a polymerization step
c) physical or chemical cross-linking of the hydrogel coating, the hydrogel forming fibers or the hydrogel matrix of a part or the whole body of the device.

In the process, the order of steps a), b) and c) may be modified or one of step b) and c) may be deleted. For example where fibers or threads or strands are coated or embedded in hydrogel, step b) is performed before step a). Step c) may be provided directly on the hydrogel forming fibers before step a) or step b) may be performed before step a).

Cross-linking in step c) may be performed by any techniques known in the art. Suitable techniques include application of heat and/or irradiation like UV or gamma rays and/or chemicals like dialdehydes in the case of PVA hydrogels and/or freezing/thawing or drying/rehydrating cycles.

In a preferred embodiment of the process in the case where polyvinyl alcohol fibers are used, step c) comprises a physical cross-linking step selected from a series of freezing /thawing cycles and/or drying/rehydrating cycles and/or a chemical cross-linking step preferably using irradiation and/or a dialdehyde cross-linker preferably glutaraldehyde.

In a third embodiment of the process according to the invention, it further comprises an annealing step of a part or the whole body of the device above the glass transition temperature of the hydrogel forming polymer and below the dissociation temperature of the hydrogel forming network. In the case of polyvinyl alcohol, the temperature of the said step is preferably in the range between 100°C to 200°C. Said annealing step may be performed directly on the fibers prior to step a) or before or after step c).

The process according to the invention the annealing step can also be performed at a temperature near the melting point of the hydrogel forming fibers, before or after step c) on a part or the whole body of the device.

In a preferred embodiment of the process, the device is maintained under tension during annealing.

According to the invention, it is possible, in the process, to treat differently and separately the end-parts and median parts in order to have condign properties for each part.

Another object of the invention is a process for preparing a device according to the present invention comprising the following steps:
a) assembly of fibers to form the body,
b) impregnation of the fibers, the assembly of fibers or a part or the whole body of the device in a solution of hydrogel forming polymer or a solution of monomer or oligomer to coat said fibers, or said strands or said assembly of fibers or said body or to embed them in a hydrogel matrix, followed in the case of a solution of monomer or oligomer by a polymerization step
c) physical or chemical cross-linking of the hydrogel coating, the hydrogel forming fibers or the hydrogel matrix of a part or the whole body of the device and, possibly, in the case where polyvinyl alcohol fibers are used
d) physical cross-linking selected from a series of freezing /thawing cycles and/or drying/rehydrating cycles and/or a chemical cross-linking step preferably using irradiation and/or a dialdehyde cross-linker preferably glutaraldehyde and, possibly,
e) annealing at a temperature near the melting point of the hydrogel forming fibers, before or after step c) of a part or the whole body of the device and possibly
f) stretching the device prior to implantation, preferably by applying a series of stretching-unstretching cycles or by applying a static stretching at constant load or strain, more preferably in a load or strain range corresponding to the physiological loads or above.

For example, in the case of human ACL, these ranges are between 0 and 2000N and between 0 and 30% for load and strain, respectively.

The device according to the invention may be used as ligament or tendon including elbow, shoulder, ankle, knee in dog, horse or human, in particular a cruciate ligament, more particularly an anterior cruciate ligament and may be configured therefore.

Consequently another object of the instant invention is a method for inserting a device comprising the following steps:
a. providing a device according to the instant invention
b. insertion of the device in the replacement site
c. attaching a first end of said device to a first attachment site
d. possibly, pretensioning the device
e. attaching the second end of the device to a second attachment site.

The first and the second attachment sites may be any musculo-skeletal tissue to which the natural ligament or tendon is attached in a human or non-human animal. In case of the anterior cruciate ligament if the first attachment site is the femur then the second attachment site is the tibia. If the first attachment site is the tibia then the second attachment site is the femur.

In order to understand the invention and to see how it may be carried out in practice, embodiments will now be described by way of examples and drawings.

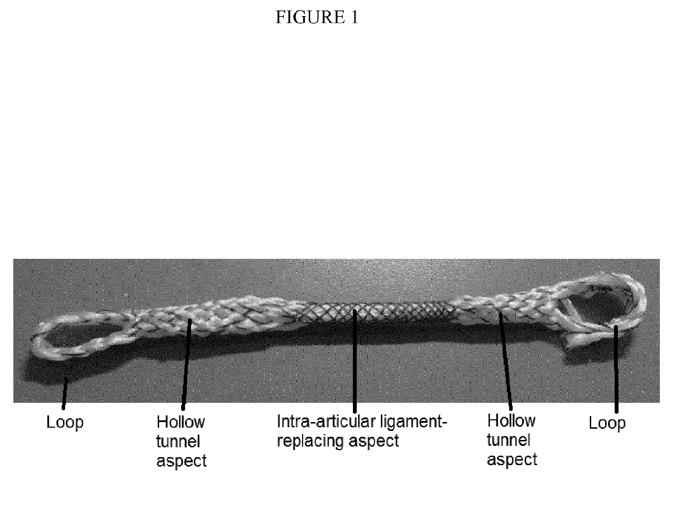

Figure 1 is the picture of one possible device obtained according to the invention. It consists of 8 PVA strands (each strand composed of 15 twisted yarns each composed of 15 twisted monofilaments, L= 80 cm) and 6 UHMWPE braided strands, each strand a 4 braid composed of many microfilaments, L=80 cm). In the median part (intra-articular ligament replacing aspect),

PVA strands are placed longitudinally in the core and UHMWPE strands form a diamond braided tubular cage. In each end parts (hollow tunnel aspects), PVA strands and UHMWPE strands are braided together to form a tunnel shaped structure. At the very extremities, two loops are formed to allow insertion of the device and/or connection to short term fixations like pins or endo-buttons.

Figure 2 illustrates the load versus strain response during a tensile test for the median part of the devices obtained following the invention with the same tubular cage structure but different sizes of the core: 8 PVA strands (8), 12 PVA strands (12) and 16 PVA strands (16).

Figure 3 illustrates the load versus strain response during a tensile test for the median part of the devices obtained following the invention with the same core structure but different braiding angles of the diamond shaped tubular cage: 40°, 45°, 50° and 55°.

### EXAMPLE 1: DEVICE DESIGN

The following examples illustrate the invention without limiting it.

Several devices have been prepared from the assembly of PVA fibers and ultra-high molecular weight polyethylene (UHMWPE) fibers.

PVA fibers (suitably with a dissolution temperature greater than 50°C and 95+% hydrolyzed) were obtained as threads of more than 600 dtex composed of several continuous fibers (for example the Solvron® MH675 from Nitivy Company Ltd). PVA strands were prepared by winding 15 PVA threads together. UHMWPE fibers were obtained as threads of more than 60dtex (for example Dyneema® from DSM).

An example of a fabrication method is given here for a device consisting of 8 PVA strands (each strand composed of 15 twisted yarns each composed of 15 twisted monofilaments, L= 80 cm) and 6 UHMWPE braided strands (each strand a 4 braid composed of many microfilaments, L=80 cm). Fabrication can be performed as follows:

First loop:
(1) The strands are divided into 4 groups as follows: 2 groups with 2 PVA strands and 2 UHMWPE strands and 2 groups with 2 PVA strands and 1 UHMWPE strand. For the first loop, each group consists of the individual strands aligned in parallel and handled as a unit.
(2) A flat 4 unit braid is applied to the central 2-3 centimeters along the length of the groups. This 4 braid forms the first loop when it is folded over and the remaining parallel loose strands on each side are brought together and aligned to be worked into the other parts of the device.

First tunnel section (adjacent to the first loop)
(3) By bringing the loose ends on each side together, the number of strands to work with is doubled to 16 of the PVA and 12 of the UHMWPE.
(4) For the tunnel aspect, the 12 UHMWPE strands are each paired with one of the PVA strands, with each pair handled as a unit. The 4 unpaired PVA strands are handled individually.
(5) The 12 paired and 4 individual PVA strands are used as components of a 16 unit, 1/1 circular diamond braid pattern applied over 3 centimeters forming the first hollow tunnel aspect.

Intra-articular ligament replacing section
(6) For the intra-articular aspect, the 12 UHMWPE strands are used to form a 12-unit, 1/1 circular braid around the 16 PVA strands aligned in parallel. This pattern is continued for another 3 centimeters.

Second tunnel section
(7) The second hollow tunnel aspect is made in the same manner as described above for the first tunnel aspect for 3 more centimeters.

Second loop
(8) The strands are divided into two groups, on opposite sides around the end of the second tunnel section, each with 8 PVA strands and 6 UHMWPE stands.
(9) Each of these groups is further divided as described above for the first loop
(10) The same 4-unit flat braid is applied to each for about 3 cm.
(11) The two braids are then joined to form the second loop.
(12) The excess strands of PVA and UHMWPE are cut and removed.

Hydrogel forming fibers can be treated by annealing. In a preferred method for PVA fibers, fiber assemblies are maintained under tension during annealing by clamping them on a metal frame and annealing is performed between 150°C and 190°C for 1h in a vacuum oven.

To embed or coat the device, a PVA solution with the desired concentration (suitably in the range 5-20wt%) was prepared either by dissolving the PVA fibers or by dissolving another PVA like PVA sold by Sigma Aldrich (suitably with MW of 80,000 to 186,000, 99+% hydrolyzed). These PVA solutions were obtained in distilled water by mixing and heating for about 1 hour around 90°C.

In one method, fiber assemblies were mounted on a metallic frame, immersed for one day at 20°C in the solution and removed from the solution. In another method, they were encased in cylindrical moulds and the PVA solution was poured in these same moulds. To cross-link the hydrogel solution, the devices obtained by these two methods were thermally cycled by freezing at about -18°C for 12 hours, and then thawing in air or distilled water by heating back to 20°C for 12 hours. This process represents one cycle by freezing-thawing. Up to 10 cycles have been applied. Cross-linking was also performed by drying the devices at 20°C in ethanol for one day and then in vacuum for one day and by rehydrating them by immersion in distilled water for one day. This process represents one cycle by drying-rehydrating.

The end parts and median part of the device could be treated separately by these two methods. In the method by immersion, only the end-parts could be embedded in the PVA matrix by partially immersing the sample. In the method with moulds, separate moulds could be used for each end- and median parts. In a first step, PVA embedding was applied to the end-parts and followed by cross-linking and possibly annealing. A second PVA embedding was then applied to the median part only or to the entire device and followed by another cross-linking step. Different stiffnesses and properties for the median and end-parts were produced by varying the degree of cross-linking achieved during the first and second steps. In one method, a solution of PVA with a dispersion of hydroxyapatite was used to coat the end-parts only.

### EXAMPLE 2: PROPERTIES OF THE DEVICE

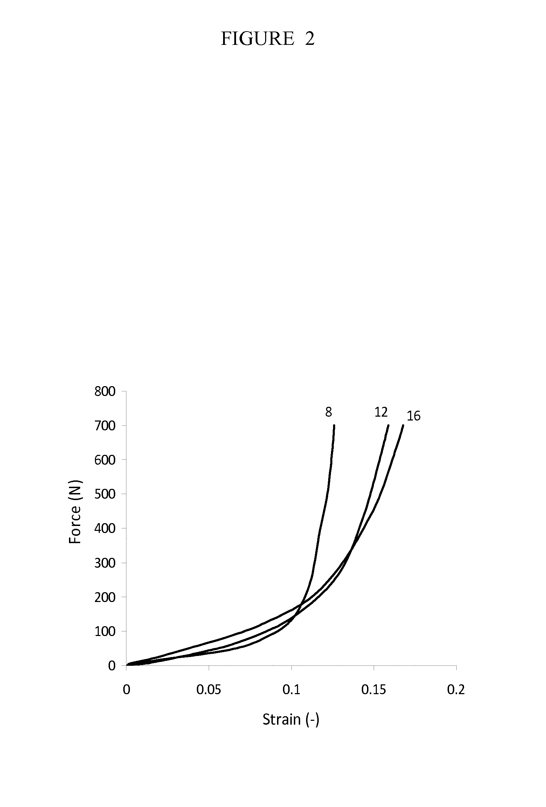

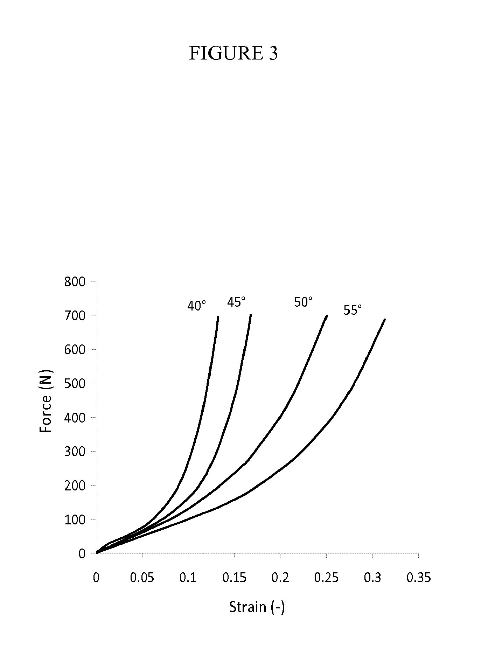

These results correspond to the fiber assembly types presented in figures 1 to 3.

### 1.1. Biocompatibility testing

1H and 13C NMR analysis showed that there is no noticeable distinction between the PVA used for the hydrogel forming fibers and a biocompatible PVA already used for cartilage replacement like the one described in US 5981826. In particular, there are no detectable traces of other organic compounds and PVA is hydrolyzed over 98%. For this latter PVA, biocompatibility was qualified by testing for ability to produce cytotoxicity, intracutaneous irritation, sensitization by Kligman maximization, Ames mutagenicity, chromosomal aberration, and chronic toxicity. Chronic toxicity was assessed in combination with long-term subcutaneous implantation in a 13-week rat animal model. The material met the acceptance criteria for all testing conducted.

### 1.2. Tensile properties

Hydrogel swelling was achieved by immersing fiber assemblies in distilled water at 23°C for 24h prior to testing. Tensile testing was performed on an Instron 5966 apparatus using capstan fixations. Samples were tested less than 5min after removal of water and pulled at a strain rate of about 10⁻²S-1 (1.5mm.s-1) which was fast enough to avoid any significant drying during testing. Testing was performed at 23°C. Instantaneous strain was measured by following ink marks with a video extensometer. Three samples were tested for each type of design.

### EXAMPLE 3: Method of measuring the tensile stiffness:

Tensile stiffness S is taken as the slope of a linear fit of the force-strain curve in the strain range 0 to 10%. Strain is measured using video extensometry by following markers placed on the sample. It is given by (L-L₀)/L₀ where L₀ and L are the initial and instantaneous distances between markers, respectively.

All measurements are made in a swollen state: samples are immerged in distilled water for over 12h prior to testing and are tested less than 5min after removal from water and pulled at a strain rate of about 10⁻²s⁻¹ (1.5mm.s⁻¹).

### EXAMPLE 4: Method of measuring the tensile behaviour in the low (0 to 5%) strain regime :

Modulus (E₀₋₅) is taken as the slope of a linear fit of the stress-strain curve in the strain range 0 to 5%. Stress is calculated by dividing the tensile force by the initial cross-section area of the median part, So. Strain is measured using video extensometry by following markers placed on the sample. It is given by (L-L₀)/L₀ where L₀ and L are the initial and instantaneous distances between markers, respectively.

All measurements are made in a swollen state: samples are immersed in distilled water for over 12h prior to testing and are tested less than 5min after removal from water and pulled at a strain rate of about 10⁻²s⁻¹ (1.5mm.s⁻¹).

### EXAMPLE 5: Method of measuring the tensile behaviour in the larger (10 to 15%) strain regime :

### Method:

Same method as for the low strain regime. Here modulus E₁₀₋₁₅ is taken as the slope of a linear fit of the stress-strain curve in the strain range 10 to 15%.

### EXAMPLE 6: Method of measuring the flexural modulus:

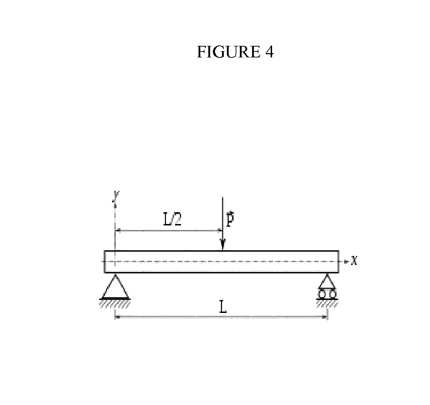

Flexural modulus E_{f} is measured by performing a 3 point-bending test under constant load P (attaching a weight in the middle of the sample), and measuring the corresponding deflection d.
E_{f} is then given by:
E_{f}= (L³/48I_{Gz})*(P/d)
where I_{Gz} = b.h³/12 for a rectangular cross-section of height h and width b.
or I_{Gz} = π.D⁴/64 for a circular cross-section of diameter D. . Figure 4

## Claims

1. A biocompatible device in the form of an elongated body comprising a flexible median part and at least one end part showing a different tensile stiffness from that of the median part, said body having a fibrous structure formed from biocompatible fibers having a diameter superior to 0.1 µm said fibers forming the body of the device wherein the median part comprises a tubular cage placed around a central core comprising fibers.

2. A device according to claim 1 in the form of an elongated body comprising a flexible median part and two end parts showing a different, preferably higher tensile stiffness from that of the median part.

3. A device according to claim 1 or 2 wherein at least part of the biocompatible fibers forming the central core of the flexible median part of the device are hydrogel forming fibers.

4. A device according to any of the claims 1 to 3 wherein the central core of the flexible median part of the device comprises further biocompatible fibers selected from biocompatible carbon fibers or metal fibers like titanium and titanium alloy fibers or polymeric fibers selected from the group consisting of polyethylene (PE), polypropylene (PP), polyamides (PA), polycarbonates (PC), polyurethanes (PU), polyurethane urea, polyesters like polyethylene terephtalate (PET), polyfluoropolymers like polytetrafluoroethylene (PTFE), polyacrylates like polymethyl methacrylate (PMMA), polyethylene glycol (PEG), poly-L-lactic acid (PLLA), poly-G-lactic acid (PGLA), poly-caprolactone (PCL), polyglycolide, polysaccharides like cellulose, hyaluronic acid, proteins like elastin, silk, collagen and from blends or copolymers obtained with polymers from this group.

5. A device according to claim 3 wherein the hydrogel forming fibers are made of polyvinyl alcohol having preferably a water absorption higher than 5% and preferably comprised between 10 and 100% in weight.

6. A device according to claim 1 wherein the tubular cage is made of fibers with a Young's modulus greater than 100MPa, preferably greater than 500MPa, made of polyethylene, preferably UHMWPE, polypropylene (PP), polyurethane (PU), polyesters like polyethylene terephthalate (PET), polyamide (PA), polyfluoropolymers like polytetrafluoroethylene (PTFE), non-polymeric fibers, preferably metallic fibers or a mixture of different fibers.

7. A device according to claim 1 wherein the tubular cage is made of hydrogel forming fibers.

8. A device according to any of the claims 1 or 8 to 9 wherein the fibers (or yarns) that form the tubular cage are woven, diamond braided or helicoidally wrapped in one or several layers around the central core of the flexible median part of the device

9. A device according to any of the claims 1 or 8 to 10 where the layers forming the tubular cage have different braiding angles, preferably where the braiding angle decreases from the innermost to the outermost layer.

10. A device according to any of the claims 1 or 8 to 11 wherein the fibers or yarns constituting the tubular cage are diamond braided or wrapped helicoidally with an angle comprised between 5 and 85°, preferably between 10 and 80°.

11. A device according to claim 1 or 2 wherein the at least one end part, preferably two end parts, are placed adjacently to at least one of the ends, preferably each of the ends of the median part and are made of swellable and porous braided, twisted, woven or knitted tunnel shaped sections

12. A device according any of the claims 1 to 13 wherein the biocompatible fibers forming the fibrous structure of the elongated body of the device are continuous.

13. A device according to any of the claims 1 or 8 to 12 wherein the woven, diamond braided or helicoidally wrapped fibers that form the tubular cage placed around the median part are continuous.

14. A device according to claim 1 wherein the continuous hydrogel forming fibers forming the body of the device are braided, twisted, knitted, woven or longitudinally disposed in the median part of the device and are braided, twisted, woven or knitted to form the tunnel shaped sections in each of the two end parts of the device.

15. A device according to claim 1 wherein the continuous fibers that are woven diamond braided or helicoidally wrapped to form the tubular cage placed around the median part of the device are braided, twisted, woven or knitted with the fibers in the central core to form the tunnel shaped sections in each of the two end parts of the device.

16. A device according to claim 1 wherein the median part shows an ultimate tensile strain greater than 6%, advantageously from 6 to 100%, more advantageously from 10 to 80%.

17. A device according to claim 1 wherein the two end parts show a tensile stiffness that is from 10% to 100 times in particular 10%, 50%, 2 or 5 times higher, than that of the flexible median part.

18. A device according to claim 1 having a median part whose tension stress-strain curve shows a low stiffness region at the lowest levels of tensile strain and a higher stiffness region at greater levels of strain.

19. A device according to claim 1 wherein the flexible median part shows in the 0 to 5% strain range and/or in the 10 to 15%. strain range a tensile modulus of 1 to 500 MPa in particular 1 to 10, 10 to 40, 40 to 100, 100 to 150, 150 to 300, 300 to 500 MPa

20. A device according to claim 1 or 10 wherein the median part shows in the 10 to 15% strain range a tensile stiffness comprised from about 10 to 500 N/%, preferably from about 20 to about 300 N/%.

21. A device according to claim 1 wherein the median part shows a flexural modulus from 0.1 to 200 MPa advantageously comprised between 1 and 100 MPa.

22. A device according to claim 1 wherein at least one end part, preferably two end parts are made of braided, twisted, woven or knitted tunnel shaped sections that contain at least one osseointegration promoting substance and/or has holes at each end of the tunnel shaped section or on the side of it to allow injection of materials into the space inside the tunnel

23. A device according to claim 1 wherein at least one end part, preferably two end parts are filled, coated and/or impregnated with a substance, preferably selected from bone mulch or bone cement or a mineral filler preferably a bioactive glass or hydroxyapatite and/or with one or several osseointegration-promoting substances, preferably calcium phosphate and/or calcium sulfate

24. A device according to claim 1 wherein the body is partly or totally coated or entrapped in a hydrogel matrix.

25. A device according to claim 1 further comprising at least at one end part an anchoring system preferably selected from the group consisting of a hook, a screw, a buckle, a bone anchor, an interference screw, a cross pin, a suture button, or an eye spliced at said end of the device.

26. Process for preparing a device according to claim 1 comprising the following steps:
a) assembly of fibers to form the body,
b) impregnation of the fibers, the assembly of fibers or a part or the whole body of the device in a solution of hydrogel forming polymer or a solution of monomer or oligomer to coat said fibers, or said strands or said assembly of fibers or said body or to embed them in a hydrogel matrix, followed in the case of a solution of monomer or oligomer by a polymerization step
c) physical or chemical cross-linking of the hydrogel coating, the hydrogel forming fibers or the hydrogel matrix of a part or the whole body of the device and, possibly, in the case where polyvinyl alcohol fibers are used
d) physical cross-linking selected from a series of freezing /thawing cycles and/or drying/rehydrating cycles and/or a chemical cross-linking step preferably using irradiation and/or a dialdehyde cross-linker preferably glutaraldehyde and, possibly,
e) annealing at a temperature near the melting point of the hydrogel forming fibers, before or after step c) of a part or the whole body of the device.
